# EUROPEAN PATENT APPLICATION

(11) **EP 1 829 484 A1**
(43) Date of publication of application: **05.09.2007**
(21) Application number: 05814169.8
(22) Date of filing: 04.11.2005
(51) Int. Cl.: A61B 10/00

(54) **DEVICE FOR COLLECTING GRAVEL AND/OR STONES DISCHARGED FROM THE URETHRA**

(30) Priority: 04.11.2004 ES 200402509 U
(71) Applicant: Fontes Muñoz, Alejandro, 30009 Murcia (ES)
(72) Inventor: Fontes Muñoz, Alejandro, 30009 Murcia (ES)
(74) Representative: Carvajal y Urquijo, Isabel
(86) International application number: PCT/ES2005/000592
(87) International publication number: WO 2006/051136

(57) **Abstract**

The present invention relates to a device which has been particularly conceived for collecting gravel and/or calculi expelled through the urethra, for example from a renal patient with kidney stones.

The object of the invention is to achieve that said calculi and/or gravel can be collected comfortably and efficiently when they are expelled while urinating, wherever the patient may be, and to learn the approximate volume of gravel expelled, and all this with a device that the patient can comfortably and discretely carry with him or her at anytime and in any place.

## Description

### DESCRIPTION OF THE INVENTION

The device proposed by the invention resolves in a fully satisfactory manner the drawbacks previously set forth in the different aspects discussed.

To that end and more specifically, said device is carried out as a type of sieve, based on a textile material with suitable porosity, finished at its lower end with a type of teat to facilitate collection, whereas at the level of its mouth, said sieve incorporates a preferably elliptical ring, carried out as a pair of rods which are in turn semi-elliptical, joined together in an articulated fashion at one of their ends, whereas at the other end they extend in straight sections for coupling to a complementary handle for holding the sieve. The articulated nature by means of which these two rods are joined together allows the folding of one over the other to minimize the volume of the device in an inoperative situation, in storage or in transport.

The straight section of one of these two rods is irremovably fixed to the handle, whereas the other one is fixed with rotational ability, having in its initial section close to the ring a flange that can be coupled in a groove of the handle or may remain outside of the latter, according to the position adopted by a moving proximal section of said handle, which is telescopically movable with respect to the rest of the handle by a magnitude that is in accordance with the length of said flange.

Locking means are arranged between the moving section of the handle and the fixed section thereof which keep said moving section perfectly stable in either of the two positions provided.

As a complement to the described structure and according to another feature of the invention, a housing is arranged in the fixed section of the handle, inside of which housing there is arranged a sample collection box, for which purpose said box has volumetric markings which allow determining the amount of sample collected, additionally having a lid for the hermetic sealing of said housing.

The device is complemented with an impermeable cover or case which allows internal housing of the duly folded device itself so that it can be carried by the user and used wherever needed.

### DESCRIPTION OF THE DRAWINGS

To complement the description being made and for the purpose of aiding to better understand the features of the invention, according to a preferred practical embodiment thereof, a set of drawings is attached as an integral part of said description, in which the following has been represented with an illustrative and nonlimiting character:
Figure 1 shows a perspective view of a device for collecting gravel and/or calculi expelled through the urethra, carried out according to the object of the present invention, which is shown in the use position and next to it an enlarged detail of the articulated joining between the two rods collaborating in the stiffening ring of the mouth of the sieve.

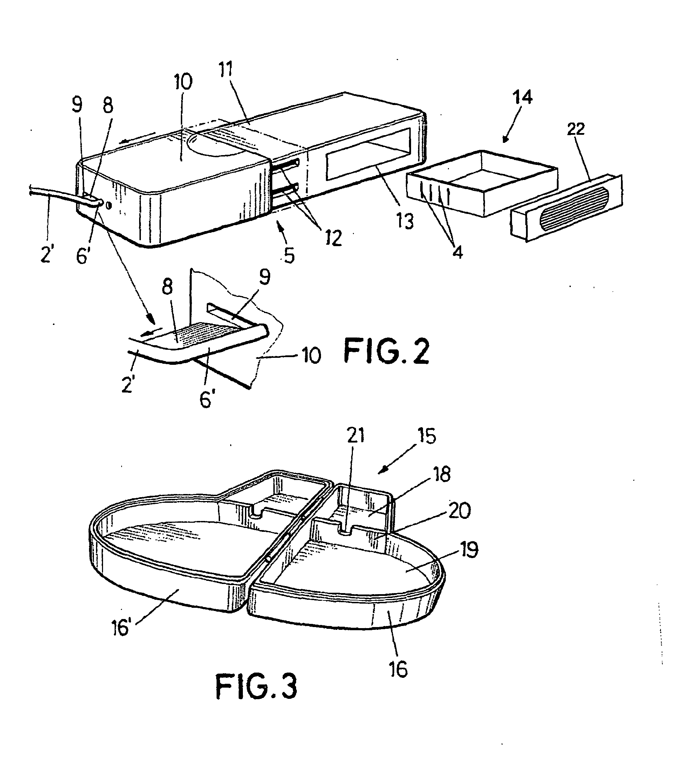

Figure 2 also shows a perspective view of a partial detail of the device, at the level of its handle, with the latter in an operative situation and with its collecting deposit being open, in which an enlarged detail of the flange and grooves for locking said ring of the mouth of the funnel have also been shown.
Figure 3 also shows a perspective view of the cover for housing the device of Figure 1, which is part of the device as a whole, which is shown in an open situation.
Figure 4 shows a plan view of the cover of the previous figure, also open, but with the collecting device itself duly housed inside it.
Finally, Figure 5 shows a plan view of the assembly of the previous figure in a closed situation.

### PREFERRED EMBODIMENT OF THE INVENTION

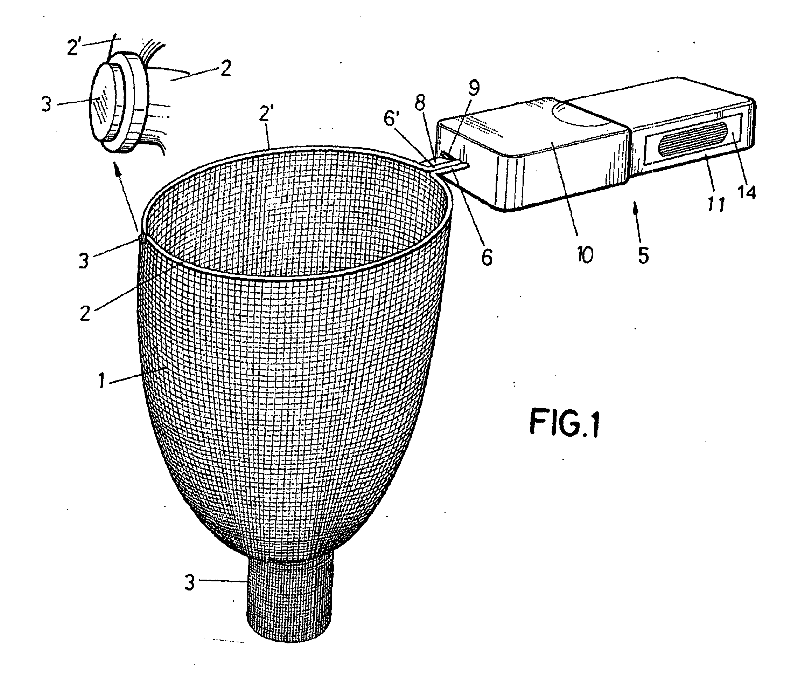

In view of the figures discussed it can be observed how the collecting device proposed by the invention is constituted of a sieve (1), of a textile nature and suitable porosity, particularly visible in Figure 1 which, due to its own textile nature, is foldable and the mouth of which is stiffened by means of a pair of essentially identical rods (2-2') which together form an ellipse with sufficient amplitude for allowing the passage therethrough of urine while the patient urinates.

The sieve (1) is finished at its lower end with a type of cylindrical teat (3) for quickly and easily collecting the amount of calculi and/or gravel expelled while urinating with a view to the adaptation thereof and subsequent supply of the corresponding data to the doctor controlling the renal patient.

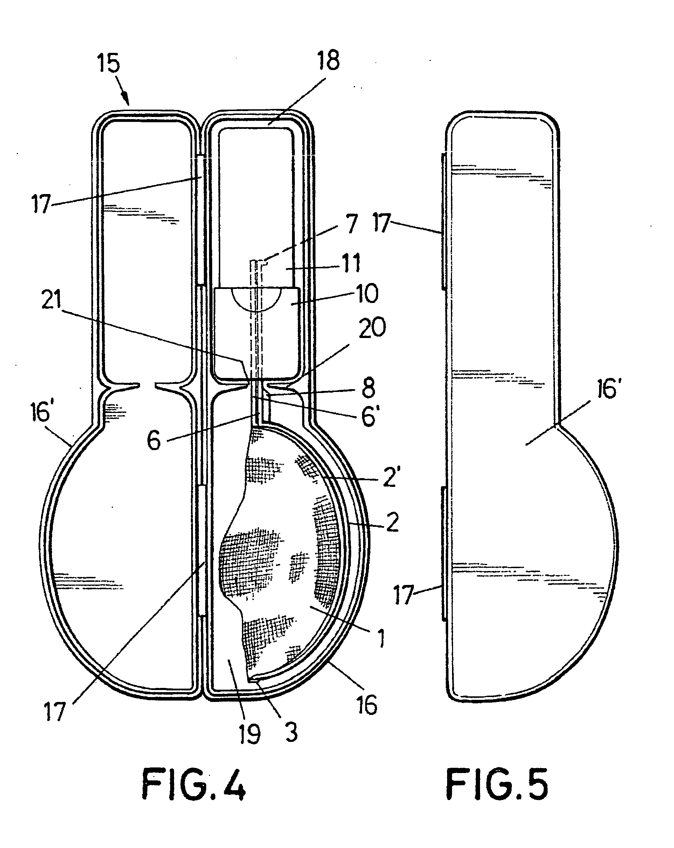

The rods (2-2') stiffening the mouth of the sieve (1) are associated to a handle (5) to facilitate handling of the device and with a view to reducing its volume in an inoperative situation, as shown in Figure 4, said rods (2-2') are joined together in an articulated fashion at one of their ends (3), as is observed particularly in the enlarged detail of Figure 1, whereas at their other end they extend in respective straight, parallel and very close sections (6-6') entering the inside of a handle (5), one of which (6) is integrally joined to said handle, without movement ability, for example through a bend (7) on its free end, whereas the other one (6') is assembled on said handle (5) with rotational ability in order to allow the folding of the ring (2-2').

For said ring (2-2') to be kept stable in an operative situation, as shown in Figure 1, that is to keep the mouth of the sieve (1) open, it has been provided that the moving rod (2'), and more specifically the area in which its curved section (2') and its straight section (6') meet, incorporates a flange (8) complementary to a groove (9) existing in the handle (5), specifically in a proximal section (10), which is telescopically movable with respect to a fixed portion (11) of said handle, said fixed portion (11) having movement guides (12) for the moving section (10) provided with safety stops locking said proximal or moving section (10) in an operative situation of the device, ensuring that the ring (2-2') stays open in such circumstances.

Finally, and as a complement of the described structure, a housing (13) is arranged in the fixed part (11) of the handle (5) for sample collection, inside of which there is arranged a sample collection box (14) which has a series of volumetric markings (4) for determining the amount of sample collected. Once the sample is collected, said box (14) is introduced in the housing (13) and said housing is subsequently hermetically sealed by means of a lid (22).

The collecting device itself, with the structure hereinbefore described, is housed in a cover or case (15) in which there is a pair of symmetrical shells (16-16'), hingedly joined together and with a single-piece nature with the cooperation of a hinge (17) and provided with any snap-fit closing means, one of said shells (16) being intended to receive the collecting device itself in a folded situation, as shown in Figure 4, and the other one is intended to constitute a sealing lid, ensuring a perfect seal for said cover. Accordingly, in this sense there is arranged in each shell (16-16') a section (18) which is suitably shaped and sized to receive the handle (5) and a section (19) in turn suitably shaped and sized to receive the ring (2-2'), with the sieve (1), duly folded, arranging an intermediate partition (20) between them which has a notch (21) on its upper edge, suitably arranged to allow passage of the rods (6-6').

## Claims

1. A device for collecting gravel and/or calculi expelled through the urethra particularly conceived for being used by renal patients and for the purpose of allowing and facilitating the collection of such residue expelled while urinating in any place and in any circumstance, **characterized in that** it is constituted by means of a type of sieve of a textile nature and suitable porosity, provided at the level of its mouth with a preferably elliptical-shaped ring, associated to a handle for handling the device, with the particularity that said ring can be folded over itself to reduce the volume of the device in an inoperative situation.

2. A device for collecting gravel and/or calculi expelled through the urethra according to claim 1, **characterized in that** the ring of the mouth of the sieve is carried out in two rods, there being defined in each one of them a curved section and a straight section, both rods being joined together in an articulated fashion at one of the ends of their curved section, whereas their straight sections enter the handle, one of them being integrally joined to the latter, whereas the other one is able to rotate in order to allow the swiveling of one half-ring with respect to the other one.

3. A device for collecting gravel and/or calculi expelled through the urethra according to claim 2, **characterized in that** the moving rod incorporates in the area where its curved section and straight sections meet, a side flange for locking said rod that can be locked into a groove of the handle, for which purpose a proximal section is arranged in the handle which is telescopically movable with respect to said handle, in which said groove is located, such that in a retracted situation of the proximal section of the handle, the flange is located outside it, the swiveling of said rod being possible, whereas in the extended position of said section the flange is locked in the groove, the fixed section of the handle further incorporating movement guides for the moving section, specifically for safety stops for the latter.

4. A device for collecting gravel and/or calculi expelled through the urethra according to any of the previous claims, **characterized in that** the sieve is finished at its lower end opposite to its mouth with a cylindrical teat which allows collecting the expelled gravel and/or calculi for their later medical control.

5. A device for collecting gravel and/or calculi expelled through the urethra according to claim 4, **characterized in that** the fixed section of the handle incorporates a housing inside of which there is arranged a sample collection box having a series of volumetric markings intended for determining the amount of sample collected, and **in that** a lid is arranged for the hermetic sealing of said housing.

6. A device for collecting gravel and/or calculi expelled through the urethra according to any of the previous claims, **characterized in that** the collecting device itself is complemented with a case formed by two shells joined by means of a hinge, able to tightly receive inside it said collecting device duly folded and to ensure a sealed closure.
